# FASCICULE DE BREVET EUROPEEN

(11) **EP 2 158 194 B1**
(45) Date de publication et mention de la délivrance du brevet: **18.05.2011**
(21) Numéro de dépôt: 08826271.2
(22) Date de dépôt: 11.06.2008
(51) Int. Cl.: C07D 401/02, C07D 471/04, C07D 403/02, A61K 31/495, A61P 35/00

(54) **DÉRIVÉS DE 7 -ALKYNYL-1,8-NAPHTHYRIDONES, LEUR PRÉPARATION ET LEUR APPLICATION EN THÉRAPEUTIQUE**
DERIVATE VON 7-ALKYNYL-1,8-NAPHTHYRIDONEN, VERFAHREN ZU IHRER HERSTELLUNG UND IHRE VERWENDUNG IN THERAPEUTIKA
DERIVATIVES OF 7-ALKYNYL-1,8-NAPHTHYRIDONES, PREPARATION METHOD THEREOF AND USE OF SAME IN THERAPEUTICS

(30) Priorité: 13.06.2007 FR 0704193
(43) Date de publication de la demande: 03.03.2010
(73) Titulaire: Sanofi-Aventis, 75013 Paris (FR)
(72) Inventeur: ALAM, Antoine, F-75013 Paris (FR); BONO, Françoise, F-75013 Paris (FR); DUCLOS, olivier, F-75013 Paris (FR); MC CORT, Gary, F-75013 Paris (FR)
(74) Mandataire: Le Coupanec, Pascale A.M.P.
(86) Numéro de dépôt international: PCT/FR2008/000794
(87) Numéro de publication internationale: WO 2009/007536

(56) Documents cités:
- WO-A-2005/118587

## Description

La présente invention se rapporte à des dérivés de 7-alkynyl-1,8-naphtyridones, à leur préparation et à leur application en thérapeutique.

Des composés pour le traitement du cancer, agissant sur VEGFR et spécialement sur VEGFR-3, sont décrit dans WO 2005/118 587.

La présente invention a pour objet des composés répondant à la formule (I) : 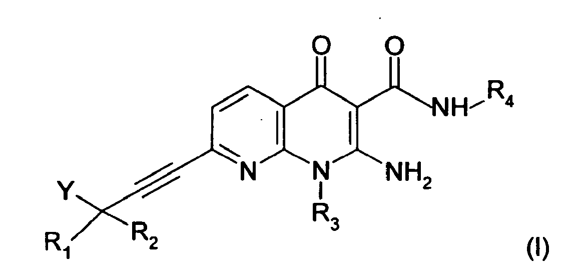 dans laquelle :
R₁ et R₂ représentent indépendamment l'un de l'autre:
   - un atome d'hydrogène,
   - un groupe alkyle en C1-C7 éventuellement substitué par un ou plusieurs groupes alcoxy,
R₃ représente un groupe alkyle en C1-C7 ;
R₄ représente un atome d'hydrogène, un groupe alkyle en C1-C4 ;
Y représente un groupe alcoxy en C1-C4, un groupe -NRR', -O(CH₂)ₙ-C(O)-NRR' où R et R' sont tels que définis ci-dessous et n est un nombre entier égal à 1 ou 2;
R" représente un groupe alkyle en C1-C4 ; et
R et R' représentent indépendamment l'un de l'autre un atome d'hydrogène, un groupe -CO-alkyle en C1 -C4 ou un groupe -COOR", où R" tel que définis ci-dessus.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leur mélange y compris leur mélange racémique font partie de l'invention.

Les composés de formule (I) peuvent exister à l'état de bases ou salifiés par des acides ou des bases, notamment des acides ou des bases pharmaceutiquement acceptables. De tels sels d'addition font partie de l'invention. Ces sels sont avantageusement préparés avec des acides ou des bases pharmaceutiquement acceptables, mais les sels d'autres acides ou de bases utiles, par exemple, pour la purification ou l'isolement des composés de formule (I), font également partie de l'invention.

Les composés selon l'invention peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, et sauf mention différente dans le texte, on entend par :
- un groupe alkyle : un groupe aliphatique saturé linéaire ou ramifié comprenant de 1 à 7 atomes de carbone (avantageusement, de 1 à 4 atomes de carbone). A titre d'exemples, on peut citer les groupes méthyle, éthyle, propyle, isopropyle, butyle, isobutyle, tertio-butyle, pentyle, hexyle, heptyle, etc ;
- un groupe alcoxy : un groupe -O-alkyle, où le groupe alkyle est tel que défini ci-dessus ;
- un atome d'halogène: un fluor, un chlore, un brome ou un iode.

Parmi les composés objet de l'invention, on peut citer un sous-groupe de composés pour lesquels Y représente un groupe alcoxy en C1-C4.

Parmi les composés objet de l'invention, on peut citer un deuxième sous-groupe de composés pour lesquels Y représente un groupe -NRR', où R et R' sont tels que définis ci-dessous.

Parmi les composés objet de l'invention, on peut citer un troisième sous-groupe de composés pour lesquels Y représente -O(CH₂)ₙ-C(O)-NRR' où R et R' sont tels que définis ci-dessous et n est un nombre entier égal à 1 ou 2.

Parmi les composés objets de l'invention, on peut notamment citer les composés suivants :
■ méthyl {3-[7-amino-8-éthyl-6-(méthylcarbamoyl)-5-oxo-5,8-dihydro-1,8-naphthyridin-2-yl]prop-2-yn-1-yl}carbamate
■ chlorhydrate de 2-amino-7-(3-amino-3-méthylbut-1-yn-1-yl)-1-éthyl-N-méthyl-4-oxo-1,4-dihydro-1,8-naphthyridine-3-carboxamide
■ (±)-2-amino-7-(3,4-diméthoxy-3-méthylbut-1-yn-1-yl)-1-éthyl-N-méthyl-4-oxo-1,4-dihydro-1,8-naphthyridine-3-carboxamide
■ 2-amino-7-[3-(2-amino-2-oxoéthoxy)-3-méthylbut-1-yn-1-yl]-1-éthyl-N-méthyl-4-oxo-1,4-dihydro-1,8-naphthyridine-3-carboxamide
■ 2-amino-1-éthyl-7-(3-méthoxyprop-1-yn-1-yl)-N-méthyl-4-oxo-1,4-dihydro-1,8-naphthyridine-3-carboxamide

Conformément à l'invention, les composés de formule (I) peuvent être préparés selon le procédé présenté dans le schéma 1.

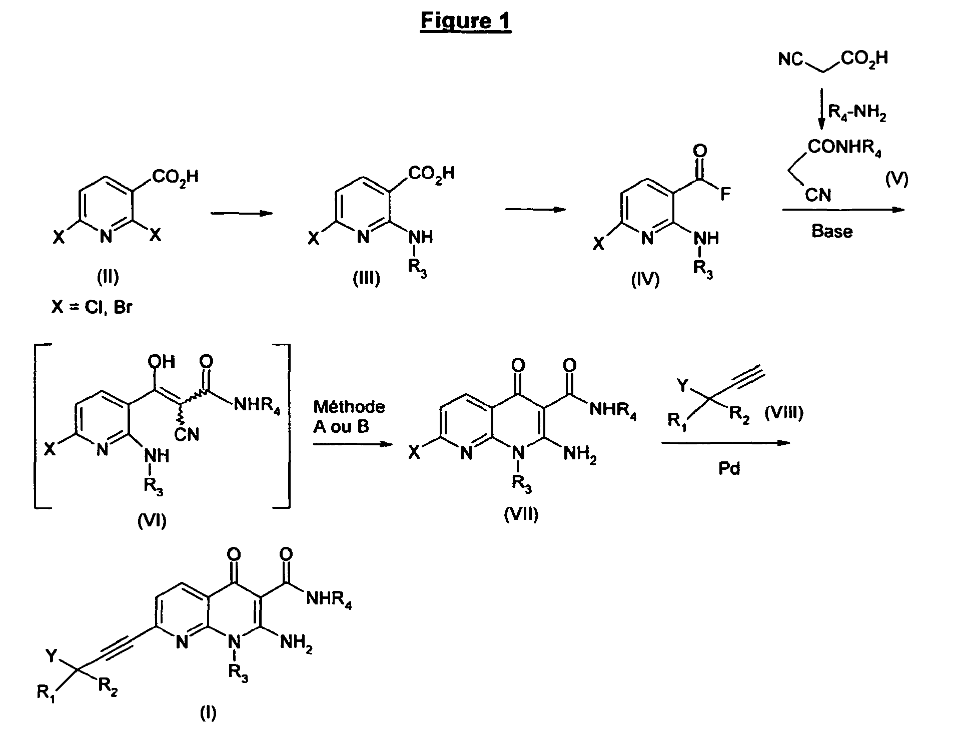

Selon la figure 1, un acide 2,6-dihalogénonicotinique de formule (II) où les groupes X représentent des atomes d'halogène (de préférence le chlore, ou le brome), et qui est soit commercial, soit préparé selon les méthodes connues de l'Homme de l'art, est monosubstitué à la position 2 par une amine de formule R₃-NH₂ (où R₃ est tel que défini précédemment en rapport avec les composés de formule (I) objets de l'invention), à une température comprise entre 20°C et 150°C, dans un solvant protique tel qu'un alcool ou l'eau et, éventuellement en tube scellé. On obtient un dérivé 2-aminonicotinique de formule (III), que l'on transforme en fluorure d'acide de formule (IV) par action du fluorure cyanuryle à température ambiante, en présence d'une base telle que la triéthylamine ou la pyridine et dans un solvant inerte tel que le dichlorométhane, comme décrit par G. OLAH et coll. dans Synthesis (1973), 487, ou par d'autres méthodes connues de l'Homme de l'art, telles que celles décrites par MUKAIYAMA et TANAKA dans Chem. Lett. (1976), 303 ou par ISHIKAWA et SASAKI dans Chem. Lett. (1976), 1407. Les fluorures d'acyles de formule (IV), très réactifs mais stables, sont mis ensuite en réaction avec un cyanoacétamide N-substitué de formule (V) en présence d'une base forte telle que l'hydrure de sodium dans un solvant polaire aprotique tel que le diméthylformamide.

Lorsque l'on emploie deux équivalents d'hydrure de sodium à l'étape de condensation du dérivé (IV) avec un dérivé (V), puis on introduit un troisième équivalent de NaH après une agitation d'entre 10 et 16 heures à température ambiante, le composé (VI) déprotonné formé se cyclise *in situ* à cette même température avec de bons rendements pour fournir directement l'amino-pyridino[2,3-b]pyridone de formule (VII) (méthode B).

Les N-alkylcyanoacétamides de formule (V) sont préparés en faisant réagir l'acide cyanoacétique avec un chloroformiate d'alkyle (tel qu'éthyle ou isobutyle) en présence d'une base telle que la triéthylamine, à une température inférieure ou égale à 0°C, puis on fait réagir l'intermédiaire anhydride mixte formé avec un excès d'amine de formule R₄-NH₂ (où R₄ est tel que défini précédemment en rapport avec les composés de formule (I) objets de l'invention).

Pour l'obtention d'une pyridino[2,3-b]pyridinone de formule (I), objet de la présente invention, l'intermédiaire halogéné de formule (VII) est couplé selon les méthodes connues de l'Homme de l'art avec un dérivé approprié d'alcool propargylique R₁R₂CH(Y)CCH de formule (VIII) où R₁, R₂ et Y sont tels que définis pour le composés de formule (I). Par exemple, l'intermédiaire (VII) est engagé dans une réaction de couplage de Sonogashira avec l'alcyne approprié de formule (VIII) en présence de PdCl₂(PPh₃)₂, d'iodure de cuivre de triéthylamine et de diméthylformamide, à une température comprise entre 80°C et 120°C. Cette réaction peut être réalisée dans un tube scellé et sous micro-ondes.

Si nécessaire au cours des étapes réactionnelles présentées dans le schéma 1, certaines fonctions réactives situées sur les groupes Y, R₁, R₂ et R₃ peuvent être protégées temporairement par des groupements protecteurs connus de l'Homme de l'art et tels que décrits dans « Protective Groups in Organic Synthesis », Green et al., 2nd Edition (John Wiley & Sons, Inc., New York).

Dans la figure 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme de l'art.

Il est également décrit les composés de formule (VII) définis dans la figure 1. Ces composés sont utiles comme intermédiaires de synthèse des composés de formule (I).

Les exemples suivants illustrent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### ■ Exemple 1 : (±)-2-amino-7-(3,4-diméthoxy-3-méthylbut-1-yn-1-yl)-1-éthyl-N-méthyl-4-oxo-1,4-dihydro-1,8-naphthyridine-3-carboxamide (composé n°1)

### 1.1 : Acide 2-(aminoéthyl)-6-chloronicotinique

On agite à température ambiante pendant 72 heures une solution de 18,0 g (84,4 mmoles) d'acide 2,6-dichloronicotinique dans 180 ml d'une solution d'éthylamine à 70 % dans l'eau. L'excès d'amine est alors évaporé sous pression réduite, puis on additionne une solution aqueuse d'acide acétique à 10 % jusqu'à la précipitation du produit. Le solide beige est essoré, rincé avec de l'eau froide et séché à l'étuve. On obtient 10,5 g du produit attendu. Point de fusion : 158-160°C. Rendement = 62 %.

### 1.2 : Fluorure d'acide 2-(aminoéthyl)-6-chloronicotinique

A une suspension de 5,0 g (24,8 mmoles) d'acide 2-(aminoéthyl)-6-chloronicotinique dans 125 ml de dichlorométhane, on ajoute 2 ml (24,8 mmoles) de pyridine et 4,2 ml (49,8 mmoles) de 2,4,6-trifluoro-triazine. On agite le mélange pendant 3 heures à température ambiante puis on filtre. Le solide est rincé avec 50 ml de dichlorométhane et le filtrat est lavé deux fois avec 60 ml d'eau glacée. On sèche la phase organique sur Na₂SO₄ et on évapore le solvant sous pression réduite. On obtient 5,01 g de produit sous forme d'huile orange. Rendement = 99 %.

### 1.3 : N-méthylcyanoacétamide

A une solution refroidie à -30°C de 10,0 g (116,38 mmoles) d'acide cyanoacétique à 99 % et 16,3 ml (116,9 mmoles) de triéthylamine dans 100 ml de THF anhydre, on ajoute goutte à goutte 12,28 ml (128,44 mmoles) de chloroformate d'éthyle puis on agite à -30°C pendant 1 heure et demie. On additionne ensuite goutte à goutte 300 ml de méthanol saturé en méthylamine gazeuse puis on agite à température ambiante pendant une nuit. On évapore les solvants sous pression réduite et on purifie le produit par filtration sur gel de silice en éluant par un mélange dichlorométhane : méthanol (95:5). On obtient 10,0 g de produit sous forme d'un solide beige. Point de fusion = 99°C. Rendement = 87 %

### Méthode A (points 1.4 et 1.5 ci-après).

### 1.4 : 3-[6-chloro-2-(éthylamino)-3-pyridinyl]-2-cyano-3-hydroxy-N-méthyl-2-propènamide

A une solution refroidie à 0-5°C de 9,80 g (100 mmoles) de N-méthylcyanoacétamide dans 100 ml de diméthylformamide anhydre, on ajoute par petites quantités 3,98 g (100 mmoles) d'hydrure de sodium à 60 % dans de l'huile minérale. Après la fin du dégagement d'hydrogène, on agite le mélange pendant 10 minutes à température ambiante, puis on le refroidit à nouveau à 0-5°C. On additionne alors une solution de 10,09 g (49,8 mmoles) de fluorure d'acide 2-(aminoéthyl)-6-chloronicotinique dans 60 ml de diméthylformamide et on agite le milieu à température ambiante pendant une nuit. On additionne 2,85 ml (49,8 mmoles) d'acide acétique et on évapore les volatiles sous pression réduite. Le résidu est repris dans de l'eau et le produit est extrait deux fois avec un mélange dichlorométhane : méthanol (95:5) puis une fois avec un mélange acétate d'éthyle : THF (2:1). Les phases organiques combinées sont séchées sur MgSO₄ puis les solvants sont évaporés sous pression réduite. On obtient 19,0 g de produit utilisé tel quel dans l'étape suivante.

### 1.5 : 2-amino-7-chloro-1-éthyl-N-méthyl-4-oxo-1,4-dihydro-1,8-naphthyridine-3-carboxamide

On chauffe pendant 48 heures à 110°C une solution de 19,0 g du produit brut obtenu à l'issue de l'étape 7.4 (49,8 mmoles) dans 600 ml de n-butanol. On évapore le solvant sous pression réduite et on triture le solide obtenu dans du méthanol. Le solide est ensuite essoré et séché à l'étuve. On obtient 7,9 g du produit attendu sous forme de solide jaune pâle. Point de fusion : 283-286°C. Rendement = 57 %.

### Méthode B (point 1.6 ci-après au lieu de 1.4 et 1.5).

### 1.6 : 2-amino-7-chloro-1-éthyl-N-méthyl-4-oxo-1,4-dihydro-1,8-naphthyridine-3-carboxamide

A une solution refroidie à 0-5 °C de 0,483 g (4,93 mmoles) de N-méthylcyanoacétamide dans 7 ml de diméthylformamide anhydre, on ajoute par petites quantités 0,394 g (9,95 mmoles) d'hydrure de sodium à 60 % dans l'huile minérale. On poursuit l'agitation à cette température pendant dix minutes puis on additionne une solution de 1,0 g (4,93 mmoles) de fluorure d'acide 2-(aminoéthyl)-6-chloronicotinique dans 5 ml de diméthylformamide. On agite le milieu pendant 1 nuit à température ambiante puis on rajoute par petites quantités 0,197 g (4,93 mmoles) d'hydrure de sodium à 60 %. On poursuit l'agitation à cette température pendant 10 minutes puis on additionne 0,56 ml (9,78 mmoles) d'acide acétique. On ajoute ensuite 60 ml d'eau et on essore le solide que l'on rince à l'eau puis on le sèche à l'étuve. On obtient 1,30 g du produit attendu. Point de fusion : 283-284°C. MH⁺= 281. Rendement = 94 %.

RMN 1H (DMSO-d6, 400MHz, , δ en ppm) : δ 11,75 (s, < 1H, très élargi); 11,00 (q, 1H, élargi); 8;45 (d, 1H); 8,10 (s, 1H élargi); 7,40 (d, 1H); 4,40 (q, 2H); 2,80 (d, 3H); 1,25 (t, 3H).

### 1.7 : Préparation du (±)-3,4-diméthoxy-3-méthylbut-1-yn-1-yl

Dans un tricol sous argon, on coule 1400 ml (0,7 mole) d'une solution commerciale de chlorure (ou bromure) d'éthynyl magnésium 0,5 M dans le tétrahydrofurane. On refroidit à 2°C avec un bain de glace et on ajoute lentement la solution de 30 g (0,327 mole) de méthoxyacétone dans 600 ml de tétrahydrofurane (exothermique). On agite pendant 1 heure à 2 °C puis on verse sur un mélange glace/NH₄Cl_{aq} saturée. On extrait à l'éther puis les phases organiques sont réunies, séchées sur sulfate de sodium, filtrées et concentrées sous vide limité. On obtient finalement le (±)-1-méthoxy-2-méthyl-3-butyn-2-ol sous forme d'une huile marron de 38 g (rendement brut quantitatif) qui est utilisée sans purification ultérieure dans l'étape suivante.

A une solution de 2 g de (±)-1-méthoxy-2-méthyl-3-butyn-2-ol (17,52 mmoles) dans 6 ml de tétrahydrofurane refroidie avec un bain de glace, on ajoute 17,5 ml de la solution de tert-butylate de potassium 1,0 M dans le tétrahydrofurane (Aldrich ; 17,52 mmoles). On agite 30 minutes à température ambiante puis on ajoute 0,55 ml d'iodure de méthyle (35,04 mmoles). On agite le mélange réactionnel à température ambiante pendant 3 heures puis on dilue à l'éther et à l'eau. Après décantation, la phase organique est lavée à l'eau, séchée sur sulfate de sodium, filtrée et concentrée sous vide limité. On obtient 2,4 g du produit attendu sous forme d'une huile jaune contenant de l'éther et du tétrahydrofurane résiduels. Le (±)-3,4-diméthoxy-3-méthylbut-1-yn-1-yl obtenu est engagé dans l'étape suivante sans purification ultérieure.

### 1.8 : (±)-2-amino-7-(3,4-diméthoxy-3-méthylbut-1-yn-1-yl)-1-éthyl-N-méthyl-4-oxo-1,4-dihydro-1,8-naphthyridine-3-carboxamide

Dans un tube pour micro-ondes de 80 ml, on place une suspension de 1,5 g (5,34 mmoles) de 2-amino-7-chloro-1-éthyl-N-méthyl-4-oxo-1,4-dihydro-[1,8]naphthyridine-3-carboxamide dans 30 ml d'un mélange DMF/Et₃N (V/V; 2/1). On fait barboter de l'argon dans cette suspension pendant 10 minutes puis on ajoute successivement 1,37 g de (±)-3,4-diméthoxy-3-méthylbut-1-yn-1-yl (10,69 mmoles), 0,101 g de Cul (0,53 mmole) et 0,187 g de bis(triphénylphosphine) palladium(II)dichloride (0,27 mmole).

On place le tube scellé dans le four à micro-ondes (appareil CEM, modèle Discover) et le mélange est chauffé sous pression à 80°C pendant 45 minutes (P = 100 W) puis refroidi et évaporé à sec. Le résidu est repris avec de l'acétate d'éthyle et de l'eau. La phase aqueuse est extraite à l'acétate d'éthyle (3 fois) puis les phases organiques sont réunies, séchées sur sulfate de sodium, filtrées et concentrées sous vide.

Le résidu obtenu est purifié par chromatographie sur colonne de silice (dépôt solide; élution avec un gradient dichlorométhane : méthanol, 100 : 0 à 98 : 2). On obtient 1,37 g du produit attendu sous forme de poudre gris pâle.

Point de fusion = 185-187°C. MH⁺ = 373. Rendement = 69 %.

RMN 1H (DMSO-d6, 400MHz, δ en ppm) : δ 11,75 (s, < 1H, très élargi); 11,00 (q, 1H, élargi); 8,45 (d, 1H); 8,00 (s, 1H élargi); 7,4 (d, 1H); 5,8 (s, 1H); 4,4 (q, 2H); 3,5-3,3 (m+s, 5H); 2,8 (d, 3H); 1,45 (s, 3H); 1,2 (t, 3H).

### ■ Exemple 2 : méthyl {3-[7-amino-8-éthyl-6-(méthylcarbamoyl)-5-oxo-5,8-dihydro-1,8-naphthyridin-2-yl]-prop-2-yn-1-yl}carbamate (composé n°2)

### 2.1 : méthyl (prop-2-yn-1-yl)carbamate

Une solution de 1,3 ml de propargylamine (18,95 mmoles, Aldrich) dans 19 ml de dioxane est refroidie à 0°C puis on ajoute 19 ml d'une solution aqueuse saturée en NaHCO₃. On agite 30 minutes à 0°C puis on additionne 1,83 ml de chloroformate de méthyle (23,68 mmoles). On agite le mélange réactionnel pendant la nuit en laissant remonter progressivement la température à l'ambiante. On extrait 4 fois à l'éther puis les phases organiques sont réunies, séchées sur sulfate de sodium, filtrées et concentrées sous vide. On obtient 1,93 g du produit attendu sous forme d'une huile jaune (rendement = 90%) que l'on utilise sans purification ultérieure.

### 2.2 : méthyl {3-[7-amino-8-éthyl-6-(méthylcarbamoyl)-5-oxo-5,8-dihydro-1,8-naphthyridin-2-yl]-prop-2-yn-1-yl}carbamate

Dans un tube pour micro-ondes de 10 ml, on place une suspension de 0,8 g (2,85 mmoles) de 2-amino-7-chloro-1-éthyl-N-méthyl-4-oxo-1,4-dihydro-[1,8]naphthyridine-3-carboxamide dans 22 ml d'un mélange DMF/Et₃N (V/V ; 2.2/1). On fait barboter de l'argon dans cette suspension pendant 10 minutes puis on ajoute successivement 0,98 g de méthyl (prop-2-yn-1-yl)carbamate (8,66 mmoles), 0,076 g de Cul (0,40 mmole) et 0,139 g de bis(triphénylphosphine) palladium(II)dichloride (0,20 mmole).

On place le tube scellé dans le four à micro-ondes (appareil CEM, modèle Discover) et le mélange est chauffé sous pression à 90°C pendant 15 minutes (P = 50 W). Après retour à l'ambiante, on évapore à sec puis on reprend avec de l'acétate d'éthyle. La phase organique est lavée successivement avec NaHCO_{3aq} puis NaCl_{aq} saturées. On sépare l'insoluble que l'on triture ensuite dans du méthanol, du tétrahydrofurane et de l'éther. On obtient 0,284 g d'un solide que l'on purifie par chromatographie sur colonne de silice (dépôt solide après solubilisation dans un mélange tétrahydrofurane/méthanol puis élution avec un gradient dichlorométhane : méthanol, 100: 0 à 98: 2). On obtient 0,095 g du produit attendu sous forme d'un solide blanc.

Point de fusion = 255 °C. MH⁺ = 357. Rendement = 9.3 %.

RMN 1H (DMSO-d6, 400MHz, δ en ppm) : δ 11,70 (s, < 1H, très élargi); 11,00 (q, 1H, élargi); 8,40 (d. 1H); 8,00 (s, 1H élargi); 7,75 (t, 1H, élargi,); 7,40 (d, 1H); 4,40 (q, 2H); 4,10 (d, 2H); 3,55 (s, 3H); 2,80 (d, 3H); 1,20 (t, 3H).

### ■ Exemple 3 : chlorhydrate de 2-amino-7-(3-amino-3-méthylbut-1-yn-1-yl)-1-éthyl-N-méthyl-4-oxo-1,4-dihydro-1,8-naphthyridine-3-carboxamide (composé n°4)

Dans un tube pour micro-ondes de 10 ml, on place une suspension de 0,84 g (3,0 mmoles) de 2-amino-7-chloro-1-éthyl-N-méthyl-4-oxo-1,4-dihydro-[1,8]naphthyridine-3-carboxamide dans 20 mL d'un mélange DMF/ Et₃N (V/V ; 7/3). On fait barboter de l'argon dans cette suspension pendant 10 minutes puis on ajoute successivement 0,50 g de 2-méthylbut-3-yn-2-amine (6,0 mmoles), 0,089 g de Cul (0,47 mmole) et 0,149 g de bis(triphénylphosphine) palladium(II)dichloride (0,21 mmole).

On place le tube scellé dans le four à micro-ondes (appareil CEM, modèle Discover) et le mélange est chauffé sous pression à 90°C pendant 15 minutes (P = 50 W) puis 25 minutes à 100°C. Après retour à l'ambiante, on évapore à sec puis on reprend avec de l'acétate d'éthyle. La phase organique est lavée successivement avec NaHCO_{3aq} puis NaCl_{aq} saturées, séchée sur sulfate de sodium, filtrée et concentrée sous vide.. Le résidu huileux obtenu est purifié par chromatographie sur colonne de silice (élution avec un gradient dichlorométhane : méthanol, 100 : 0 à 97 : 3). On obtient 0,151 g (0,46 mmole) du produit attendu que l'on met en solution dans 3 ml d'acétate d'éthyle puis on ajoute 0,13 ml (0,52 mmole) d'une solution de dioxane chlorhydrique 4N. Après filtration et séchage à l'étuve sous vide, on obtient 0,134 mg de produit attendu sous forme d'un solide jaune.

Point de fusion = 293 °C. MH⁺ = 310. Rendement = 12 %.

RMN 1H (DMSO-d6, 400MHz, δ en ppm) : δ 11.75 (s, < 1H, très élargi), 11.00 (q, 1H, élargi), 8.40 (d, 1H), 8.10 (s, < 1H, élargi), 7.45 (d, 1H), 4.40 (q, 2H), 3.50 (dd, 2H), 3.35 (s, 6H), 2.85 (d, 3H), 1.45 (s, 3H), 1.20 (t, 3H).

### Exemple 4: 2-amino-7-[3-(2-amino-2-oxoéthoxy)-3-méthylbut-1-yn-1-yl]-1-éthyl-N-méthyl-4-oxo-1,4-dihydro-1,8-naphthyridine-3-carboxamide (composé n°6)

### 4.1 : [(1,1-diméthylprop-2-yn-1-yl)oxy]acétate d'éthyle

Une solution de 0,87 g (10,32 mmoles) de 2-méthylbut-3-yn-2-ol dans 20 ml de THF anhydre est refroidie à 0-5°C avec un bain de glace puis on ajoute 10,32 ml (10,32 mmoles) de t-butylate de potassium 1,0 M dans le THF (Aldrich). On agite 10 minutes à froid puis on ajoute 1,89 g (11,35 mmoles) de bromoacétate d'éthyle. On agite le mélange réactionnel pendant 45 minutes à température ambiante puis on ajoute HClaq 0,1N et de l'éther. La phase organique est séchée sur sulfate de sodium, filtrée et concentrée sous vide. On obtient 1,45 g du produit attendu sous forme d'une huile jaune que l'on utilise sans purification ultérieure.

Rendement = 83 %.

### 4.2 : ({3-[7-amino-8-éthyl-6-(méthylcarbamoyl)-5-oxo-5,8-dihydro-1,8-naphthyridin-2-yl]-1,1-diméthylprop-2-yn-1-yl}oxy)acétate d'éthyle

On place 1,15 g (4,10 mmoles) de 2-amino-7-chloro-1-éthyl-N-méthyl-4-oxo-1,4-dihydro-[1,8]naphthyridine-3-carboxamide et 1,39 g (8,19 mmoles) de [(1,1-diméthylprop-2-yn-1-yl)oxy]acétate d'éthyle dans un mélange de 10 ml de diméthylformamide et 10 ml de triéthylamine. On fait barboter de l'argon pendant 15 minutes dans le mélange réactionnel puis on ajoute successivement 0,031 g (0,16 mmole) de Cul et 0,144 g (0,20 mmole) de bis(triphénylphosphine) palladium(II)dichloride Le mélange réactionnel est chauffé pendant 15 heures à 90 °C. Après retour à température ambiante, le mélange réactionnel est versé sur un mélange eau/glace. Après décantation, la gomme noire obtenue est solubilisée dans l'acétate d'éthyle puis lavée à l'eau. La phase organique est séchée sur sulfate de sodium, filtrée et concentrée sous vide. On obtient 1,4 g d'une huile marron que l'on purifie par chromatographie sur silice (élution avec un gradient cyclohexane/acétate d'éthyle, 30: 70 à 20 :80) pour donner 0,72 g de produit attendu sous forme d'un solide jaune.

Rendement = 41 %.

### 4.3 : 2-amino-7-[3-(2-amino-2-oxoéthoxy)-3-méthylbut-1-yn-1-yl]-1-éthyl-N-méthyl-4-oxo-1,4-dihydro-1,8-naphthyridine-3-carboxamide

Dans un tube scellé de 50 ml, on place 0,30 g (0,72 mmole) de ({3-[7-amino-8-éthyl-6-(méthylcarbamoyl)-5-oxo-5,8-dihydro-1,8-naphthyridin-2-yl]-1,1-diméthylprop-2-yn-1-yl}oxy)acétate d'éthyle dans 30 ml de méthanol. On refroidit la solution avec un bain de glace et on fait barboter de l'ammoniaque gazeux jusqu'à saturation. On chauffe pendant 8 heures 80°C puis on évapore à sec. Le résidu obtenu est purifié par chromatographie sur colonne de silice (dépôt solide après solubilisation dans un mélange tétrahydrofurane/méthanol puis élution avec un gradient dichlorométhanelméthanol, 100: 0 à 95:5) pour donner 0,18 g de produit attendu sous forme d'un solide blanc.

Point de fusion = 204°C. MH⁺ = 386. Rendement = 64 %.

RMN 1H (DMSO-d6, 400MHz, δ en ppm) : δ 11,85 (s, < 1H, très élargi); 11,00 (q, 1H, élargi); 8,45 (d, 1H); 7,45 (d, 1H); 7,20 (d, < 2H, élargi), 4,40 (q, 2H), 3,95 (s, 2H); 2,80 (d, 3H);1,60 (s, 6H), 1,20 (t, 3H).

Le tableau qui suit illustre les structures chimiques et les propriétés physiques de quelques composés de formule (I) selon l'invention. Dans ce tableau :
- Me et Et représentent respectivement des groupes méthyle et éthyle,
- dans la colonne « sel », « - » représente un composé sous forme de base libre, alors que « HCl » représente un composé sous forme de chlorhydrate,
- la colonne PF indique le point de fusion, en °C, du composé, et
- dans la colonne CUSM, sont indiqués successivement, la méthode analytique de chromatographie liquide haute performance utilisée (A ou B) et détaillée ci-dessous, le temps de rétention du composé exprimé en minute, et le pic MH⁺ identifié par spectrométrie de masse.

| | |
|---|---|
| | .Méthode A : |
| Colonne : | Gemini, 50x3 mm, 3µm |
| Solvant A : | H₂O + 0.1% HCO₂H; solvant B : ACN + 0.1% HCO₂H; débit = 1 mUmn |
| Gradient : | 95/5 (0 min) to 0/100 (5,5 min) to 0/100 (7,5 min) |
| Détection : | 220 nM |
| lonisation : | ESI+ |
| | .Méthode B : |
| Colonne : | Kromasil, 50x2.1 mm, 3,5 µm |
| Solvant A : | CH3CO2NH4 5 mM; solvant B : ACN; débit = 0,5 mL/mn |
| Gradient : | 100/0 (0 min) to 0/100 (13 min) to 0/100 (16 min) |
| Détection : | 220 nM |
| lonisation : | ESI+ |

- dans la colonne chiralité, « / » représente un composé achiral et (±) représente un composé sous forme de mélange racémique.

**Tableau**

| | | | | | | | | | |
|---|---|---|---|---|---|---|---|---|---|
| 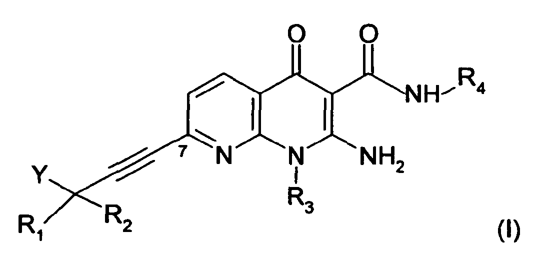 | | | | | | | | | |

| **N°** | **R1** | **R2** | **R3** | **R4** | **Y** | **Sel** | **CL/SM** | **PF** | **Chiralité** |
|---|---|---|---|---|---|---|---|---|---|
| **1** | Me | 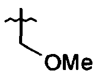 | Et | Me | OMe | - | B 6.16 373 | 185-187 | (±) |
| **2** | H | H | Et | Me | 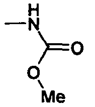 | - | B 3.48 358 | 250 | / |
| **3** | H | H | Et | Me | 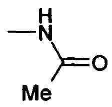 | - | C 4.94 342 | 239 | / |
| **4** | Me | Me | Et | Me | NH₂ | HCl | B 4.08 327 | 293 | / |
| **5** | H | H | Et | Me | OMe | - | C 6.8 315 | 233 | / |
| **6** | Me | Me | Et | Me | 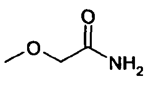 | - | B 3.83 386 | 204 | / |

Les composés selon l'invention ont fait l'objet d'essais pharmacologiques permettant de déterminer leur effet inhibiteur de l'enzyme VEGFR-3.

### Mesure de l'activité tyrosine kinase du VEGFR-3 par ELISA

L'activité enzymatique de VEGFR-3 est évaluée sur un test ELISA par mesure de l'intensité de phosphorylation du substrat poly Glu-Tyr. L'effet des produits est quantifié par la concentration qui diminue l'activité totale de l'enzyme de 50% (IC50). Pour la détermination des IC50, le produit est dilué dans du DMSO avec une gamme de concentration qui s'étale de 3 à 1000 nM. La veille de la manipulation, 125µl du substrat poly Glu-Tyr (250 µg/ml en PBS 1x sans Ca²⁺ ni Mg²⁺ ni sodium bicarbonate), sont déposés dans chaque puit d'une plaque ELISA (par exemple une plaque ELISA du kit SIGMA Protein Tyrosine Kinase Assay, Ref. PTK-101). La plaque est ensuite recouverte par un adhésif et incubée une nuit à 37°C. Le lendemain, les puits sont vidés par retournement, lavés par ajout de 300 µl de solution tampon (PBS + 0,05% Tween 20) et séchés par une nouvelle incubation de la plaque pendant 2 h à 37°C. Un mélange réactionnel de 90 µl est déposé sur chaque puits. Ce mélange contient le tampon kinase 1X additionné de 30µM d'ATP et de l'inhibiteur à la concentration souhaitée. Ensuite, 20 µl de VEGFR-3-TK (Cell signaling, Ref. 7790), préalablement diluée dans le tampon kinase sans ATP, seront additionnés (à l'exception des puits contrôle négatif où 20 µl de tampon sans enzyme seront rajoutés). Les plaques sont ensuite incubées sous agitation douce à température ambiante pendant 30 min. Après 3 rinçages avec la solution tampon (300µl / puits par lavage), 100 µl d'anticorps anti-Phospho tyrosine-HRP (1 / 30 000). Sont ajoutés à chaque puits et de nouveau les plaques sont Incubées pendant 30 min à température ambiante sous agitation douce. Après 3 lavages en solution tampon (300µl / puits par lavage), la phosphorylation du substrat est révélée par l'addition de 100 µl par puits de substrat OPD, 1 pastille OPD et 1 pastille urée dans 20 ml d'eau (préparation extemporanée et à l'abri de la lumière). Après incubation de 7 minutes à température ambiante et à l'abri de la lumière la réaction est arrêtée par ajout de 100 µl H₂S0₄ à 1,25 M (2,5N) par puit et l'absorbance est lue à 492 nm. L'activité totale est évaluée par la différence de densité optique obtenue sur des échantillons incubés en présence (stimulé) et en absence (non-stimulé) de VEGFR-3.

Les composés conformes à l'invention présentent des CI50 inferieures à 10 µM, pour la plupart inférieures à 1 µM. A titre d'exemples, les composés n°4 et 5 du tableau présentent des CI50 de 451 nM et 343 nM, respectivement.

Il apparaît donc que les composés selon l'invention ont une activité inhibitrice de l'enzyme VEGFR-3 ; ils peuvent donc être utilisés pour la préparation de médicaments, en particulier de médicaments inhibiteurs de VEGFR-3.

Ainsi, selon un autre de ses aspects, l'invention a pour objet des médicaments qui comprennent un composé de formule (I), ou un sel d'addition de ce dernier à un acide ou une base pharmaceutiquement acceptable, ou encore un hydrate ou un solvat ainsi qu'un énantiomère ou un diastéréoisomère y compris leur mélange du composé de formule (I).

Un autre aspect de l'invention comprend une association entre au moins un composé selon l'invention et au moins un agent de chimiothérapie.

En effet, les composés de la présente invention peuvent être utilisés seuls ou en mélange avec au moins un agent de chimiothérapie pouvant être choisi parmi :
● les agents alkylants,
● les agents intercalants,
● les agents antimicrotubules,
● les agents antimitotiques,
● les agents antimétabolites,
● les agents anti-prolifératifs,
● les agents antibiotiques,
● les agents immunomodulateurs,
● les agents anti-inflammatoires,
● les inhibiteurs de kinases,
● les agents anti-angiogèniques,
● les agents antivasculaires,
● les hormones oestrogéniques et androgéniques,
et les prodrogues des agents ou dérivés mentionnés ci-dessus.

Il est également possible de combiner les composés selon l'invention avec un traitement par radiations.

Les combinaisons des composés de l'invention avec les agents de chimiothérapie cités ci-dessus et/ou les radiations sont un autre objet de la présente invention.

Les agents de chimiothérapie cités ci-dessus et/ou les radiations peuvent être administrés simultanément, séparément ou séquentiellement. Le traitement sera adapté par le praticien en fonction du malade à traiter.

Ces médicaments trouvent leur emploi en thérapeutique, notamment dans le traitement et la prévention :
- des cancers et de leurs métastases, tels que : glioblastomes, myélomes multiples, syndromes myélodysplasiques, sarcomes de Kaposi, tumeurs solides, lymphomes, mélanomes, cancers du sein, cancers colorectaux, cancers des poumons, y compris les cancers non à petites cellules, cancers du pancréas, cancers de la prostate, cancers rénaux, cancers de la tête et du cou, cancer du foie, cancers des ovaires, cancers de l'appareil respiratoire et thoracique, angiogénèses tumorales, autres tumeurs exprimant VEGFR-3 ou impliquant un processus d'angiogenèse ou de lymphangiogenèse
- des maladies prolifératives non oncologiques et des angiogénèses pathologiques liées au VEGFR-3, telles que : arthroses, resténoses, psoriasis, hémangiomes, glaucomes, glomérulonéphrites, néphropathies diabétiques, néphroscléroses, syndromes microangiopathiques thrombotiques, cirrhoses du foie, athéroscléroses, rejets de greffe d'organe, des maladies de l'oeil impliquant u processus d'angiogénèse ou de lymphangiogénèse telles que la rétinopathie diabétique ou la dégénérescence maculaire,
- ou encore dans le traitement et la prévention de l'inflammation (chronique ou non), de l'infection par les microorganismes et des maladies auto-immunes, telle que la polyarthrite rhumatoïde,
- ou encore dans le traitement de maladies rares telles que la lymphangioleiomyomatose.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intraveineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour le traitement ou la prévention des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) 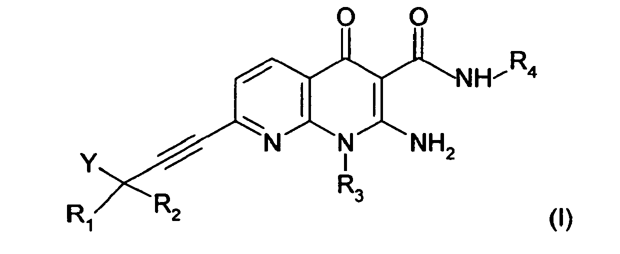 dans laquelle :
R₁ et R₂ représentent indépendamment l'un de l'autre:
. un atome d'hydrogène,
. un groupe alkyle en C1-C7 éventuellement substitué par un ou plusieurs groupes alcoxy,
R₃ représente un groupe alkyle en C1-C7 ;
R₄ représente un atome d'hydrogène, un groupe alkyle en C1-C4 ;
Y représente un groupe alcoxy en C1-C4, un groupe -NRR', -O(CH₂)ₙ-C(O)-NRR' où R et R' sont tels que définis ci-dessous et n est un nombre entier égal à 1 ou 2;
R" représente un groupe alkyle en C1-C4 ; et
R et R' représentent indépendamment l'un de l'autre un atome d'hydrogène, un groupe -CO-alkyle en C1 -C4 ou un groupe -COOR", où R" tel que définis ci-dessus,
à l'état de base ou de sel d'addition à un acide, ainsi qu'un énantiomère ou un diastéréoisomère y compris leur mélange.

2. Composés selon la revendication 1, pour lesquels pour lesquels Y représente un groupe alcoxy en C1-C4,
à l'état de base ou de sel d'addition à un acide, ainsi qu'un énantiomère ou un diastéréoisomère y compris leur mélange.

3. Composés selon la revendication 1, pour lesquels pour lesquels Y représente un groupe -NRR', où R et R' sont tels que dans la revendication 1,
à l'état de base ou de sel d'addition à un acide, ainsi qu'un énantiomère ou un diastéréoisomère y compris leur mélange.

4. Composés selon la revendication 1, pour lesquels pour lesquels Y représente un groupe -O(CH₂)ₙ-C(O)-NRR', où R et R' sont tels que dans la revendication 1 et n est un nombre entier égal à 1 ou 2,
à l'état de base ou de sel d'addition à un acide, ainsi qu'un énantiomère ou un diastéréoisomère y compris leur mélange.

5. Composés selon la revendication 1, **caractérisé en ce qu'**il est choisi parmi :
■ méthyl {3-[7-amino-8-éthyl-6-(méthylcarbamoyl)-5-oxo-5,8-dihydro-1,8-naphthyridin-2-yl]prop-2-yn-1-yl}carbamate
■ chlorhydrate de 2-amino-7-(3-amino-3-méthylbut-1-yn-1-yl)-1-éthyl-N-méthyl-4-oxo-1,4-dihydro-1,8-naphthyridine-3-carboxamide
■ (±)-2-amino-7-(3,4-diméthoxy-3-méthylbut-1-yn-1-yl)-1-éthyl-N-méthyl-4-oxo-1,4-dihydro-1,8-naphthyridine-3-carboxamide
■ 2-amino-7-[3-(2-amino-2-oxoéthoxy)-3-méthylbut-1-yn-1-yl]-1-éthyl-N-méthyl-4-oxo-1,4-dihydro-1,8-naphthyridine-3-carboxamide
■ 2-amino-1-éthyl-7-(3-méthoxyprop-1-yn-1-yl)-N-méthyl-4-oxo-1,4-dihydro-1,8-naphthyridine-3-carboxamide.

6. Procédé de préparation d'un composé de formule (I) selon l'une quelconque des revendications 1 à 5, **caractérisé en ce que** l'on fait réagir un composé de formule (VII) 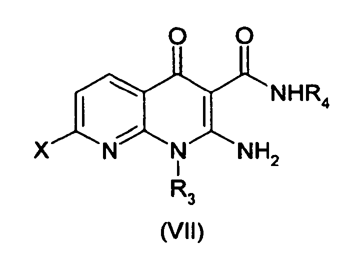 dans laquelle X est un atome d'halogène et R₃ et R₄ sont tels que définis dans l'une des revendications précédentes
avec un composé de formule (VIII) 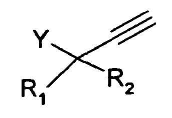 dans laquelle Y, R₁ et R₂ sont tels que définis dans l'une des revendications précédentes

7. Médicament, **caractérisé en ce qu'**il comprend un composé de formule (I) selon l'une quelconque des revendications 1 à 5, ou un sel d'addition de ce composé à un acide pharmaceutiquement acceptable, ou encore un énantiomère, un diastéréoisomère ou leur mélange du composé de formule (I).

8. Composition pharmaceutique, **caractérisée en ce qu'**elle comprend un composé de formule (I) selon l'une quelconque des revendications 1 à 5, ou un sel pharmaceutiquement acceptable, ou encore un énantiomère, un diastéréoisomère ou leur mélange de ce composé, ainsi qu'au moins un excipient pharmaceutiquement acceptable.

9. Association d'au moins un composé de formule (I) selon l'une quelconque des revendications 1 à 5 avec au moins un agent de chimiothérapie choisi parmi :
● les agents alkylants,
● les agents intercalants,
● les agents antimicrotubules,
● les agents antimitotiques,
● les agents antimétabolites,
● les agents anti-prolifératifs,
● les agents antibiotiques,
● les agents immunomodulateurs,
● les agents anti-inflammatoires,
● les inhibiteurs de kinases,
● les agents anti-angiogèniques,
● les agents antivasculaires,
● les hormones oestrogéniques et androgéniques.

10. Utilisation d'un composé de formule (I) selon l'une quelconque des revendications 1 à 5 pour la préparation d'un médicament destiné au traitement de toute maladie dans laquelle le VEGFR-3 est impliqué.

11. Utilisation d'un composé de formule (I) selon l'une quelconque des revendications 1 à 5 pour la préparation d'un médicament destiné au traitement et/ou à la prévention des cancers et de leurs métastases.

12. Utilisation d'un composé de formule (I) selon la revendication 11 pour la préparation d'un médicament destiné au traitement et/ou à la prévention des glioblastomes, myélomes multiples, syndromes myélodysplasiques, sarcomes de Kaposi, angiosarcomes cutanés, tumeurs solides, lymphomes, mélanomes, cancers du sein, cancers colorectaux, cancers des poumons, y compris les cancers non à petites cellules, cancers du pancréas, cancers de la prostate, cancers rénaux, cancers de la tête et du cou, cancer du foie, cancers des ovaires, cancers de l'appareil respiratoire et thoracique, autres tumeurs exprimant VEGFR-3 ou impliquant un processus d'angiogénèse ou de lymphangiogénèse.

13. Utilisation d'un composé de formule (I) selon l'une quelconque des revendications 1 à 5 pour la préparation d'un médicament destiné au traitement et/ou à la prévention des maladies prolifératives non oncologiques et des angiogenèses pathologiques liées au VEGFR-3.

14. Utilisation d'un composé de formule (I) selon la revendication 13 pour la préparation d'un médicament destiné au traitement et/ou à la prévention des telles que : arthroses, resténoses, psoriasis, hémangiomes, lymphangiomes, glaucomes, glomérulonéphrites, néphropathies diabétiques, néphroscléroses, syndromes microangiopathiques thrombotiques, cirrhoses du foie, athéroscléroses, rejets de greffe d'organe, des maladies de l'oeil impliquant un processus d'angiogénèse ou de lymphangiogénèse,

15. Utilisation d'un composé de formule (I) selon l'une quelconque des revendications 1 à 5 pour la préparation d'un médicament destiné au traitement et/ou à la prévention de l'inflammation chronique ou non, de l'infection par les microorganismes et des maladies auto-immunes, telle que la polyarthrite rhumatoïde.

16. Utilisation d'un composé de formule (I) selon l'une quelconque des revendications 1 à 5 pour la préparation d'un médicament destiné au traitement et/ou à la prévention de maladies rares telles que la lymphangioleiomyomatose.

## Claims

1. Compound corresponding to the formula (I): 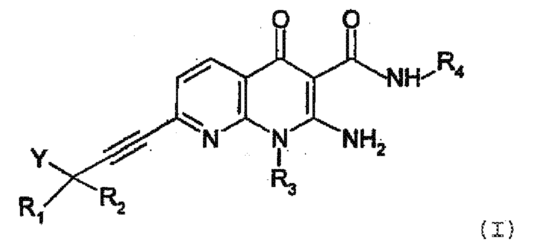 in which:
R₁ and R₂ represent, independently of one another:
● a hydrogen atom,
● a C₁-C₇ alkyl group optionally substituted by one or more alkoxy groups,
R₃ represents a C₁-C₇ alkyl group,
R₄ represents a hydrogen atom or a C₁-C₄ alkyl group,
Y represents a C₁-C₄ alkoxy group or an -NRR' or -O(CH₂)ₙ-C(O)-NRR' group where R and R' are as defined below and n is an integer equal to 1 or 2,
R'' represents a C₁-C₄ alkyl group, and
R and R' represent, independently of one another, a hydrogen atom, a -CO-(C₁-C₄ alkyl) group or a -COOR'' group, where R'' is as defined above,
in the form of the base or of an addition salt with an acid, and an enantiomer or a diastereoisomer, including their mixture.

2. Compound according to Claim 1, for which Y represents a C₁-C₄-alkoxy group,
in the form of the base or of an addition salt with an acid, and an enantiomer or a diastereoisomer, including their mixture.

3. Compound according to Claim 1, for which Y represents an -NRR' group where R and R' are as in claim 1,
in the form of the base or of an addition salt with an acid, and an enantiomer or a diastereoisomer, including their mixture.

4. Compound according to Claim 1, for which Y represents an -O(CH₂)ₙ-C(O)-NRR' group where R and R' are as in claim 1 and n is an integer equal to 1 or 2, in the form of the base or of an addition salt with an acid, and an enantiomer or a diastereoisomer, including their mixture.

5. Compound according to Claim 1, **characterized in that** it is chosen from:
■ methyl {3-[7-amino-8-ethyl-6-(methylcarbamoyl)-5-oxo-5,8-dihydro-1,8-naphthyridin-2-yl]prop-2-yn-1-yl}carbamate
■ 2-amino-7-(3-amino-3-methylbut-1-yn-1-yl)-1-ethyl-N-methyl-4-oxo-1,4-dihydro-1,8-naphthyridine-3-carboxamide hydrochloride
■ (±)-2-amino-7-(3,4-dimethoxy-3-methylbut-1-yn-1-yl)-1-ethyl-N-methyl-4-oxo-1,4-dihydro-1,8-naphthyridine-3-carboxamide
■ 2-amino-7-[3-(2-amino-2-oxoethoxy)-3-methylbut-1-yn-1-yl]-1-ethyl-N-methyl-4-oxo-1,4-dihydro-1,8-naphthyridine-3-carboxamide
■ 2-amino-1-ethyl-7-(3-methoxyprop-1-yn-1-yl)-N-methyl-4-oxo-1,4-dihydro-1,8-naphthyridine-3-carboxamide.

6. Process for the preparation of a compound of formula (I) according to any one of Claims 1 to 5, **characterized in that** a compound of formula (VII) 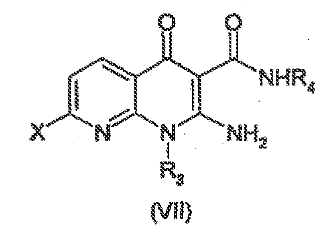 in which X is a halogen atom and R₃ and R₄ are as defined in one of the preceding claims,
is reacted with a compound of formula (VIII) 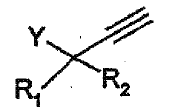 in which Y, R₁ and R₂ are as defined in one of the preceding claims.

7. Medicament, **characterized in that** it comprises a compound of formula (I) according to any one of Claims 1 to 5, or an addition salt of this compound with a pharmaceutically acceptable acid, or also an enantiomer, a diastereoisomer or their mixture of the compound of formula (I).

8. Pharmaceutical composition, **characterized in that** it comprises a compound of formula (I) according to any one of Claims 1 to 5, or a pharmaceutically acceptable salt, or also an enantiomer, a diastereoisomer or their mixture of this compound, and also at least one pharmaceutically acceptable excipient.

9. Combination of at least one compound of formula (I) according to any one of Claims 1 to 5 with at least one chemotherapy agent chosen from:
● alkylating agents,
● intercalating agents,
● antimicrotubule agents,
● antimitotic agents,
● antimetabolites,
● antiproliferative agents,
● antibiotics,
● immunomodulatory agents,
● anti-inflammatories,
● kinase inhibitors,
● antiangiogenic agents,
● antivascular agents,
● estrogenic and androgenic hormones.

10. Use of a compound of formula (I) according to any one of Claims 1 to 5 in the preparation of a medicament intended for the treatment of any disease in which VEGFR-3 is involved.

11. Use of a compound of formula (I) according to any one of Claims 1 to 5 in the preparation of a medicament intended for the treatment and/or prevention of cancers and metastases thereof.

12. Use of a compound of formula (I) according to Claim 11 in the preparation of a medicament intended for the treatment and/or prevention of glioblastomas, multiple myelomas, myelodysplasic syndromes, Kaposi's sarcomas, cutaneous angiosarcomas, solid tumors, lymphomas, melanomas, breast cancers, colorectal cancers, lung cancers, including non-small-cell cancers, pancreatic cancers, prostate cancers, kidney cancers, head and neck cancers, liver cancers, ovarian cancers, cancers of the respiratory tract and chest, other tumors expressing VEGFR-3 or involving a process of angiogenesis or of lymphangiogenesis.

13. Use of a compound of formula (I) according to any one of Claims 1 to 5 in the preparation of a medicament intended for the treatment and/or prevention of non-oncological proliferative diseases and pathological angiogenesis linked to VEGFR-3.

14. Use of a compound of formula (I) according to Claim 13 in the preparation of a medicament intended for the treatment and/or prevention of such diseases as: arthroses, restenoses, psoriasis, hemangiomas, lymphangiomas, glaucomas, glomerulonephritis, diabetic nephropathies, nephrosclerosis, thrombotic microangiopathic syndromes, liver cirrhosis, atherosclerosis, organ transplant rejection, or eye diseases involving a process of angiogenesis or of lymphangiogenesis.

15. Use of a compound of formula (I) according to any one of Claims 1 to 5 in the preparation of a medicament intended for the treatment and/or prevention of chronic or non-chronic inflammation, of infection by microorganisms and of autoimmune diseases, such as rheumatoid arthritis.

16. Use of a compound of formula (I) according to any one of Claims 1 to 5 in the preparation of a medicament intended for the treatment and/or prevention of rare diseases, such as lymphangioleiomyomatosis.

## Patentansprüche

1. Verbindung der Formel (I) 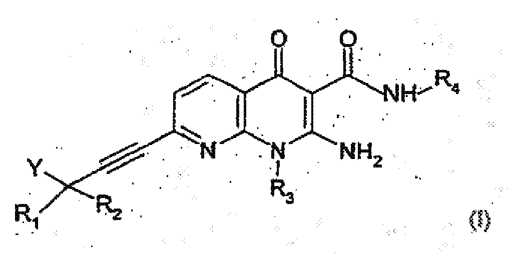 worin:
R1 und R₂ unabhängig voreinander für
. ein Wasserstoffatom,
. oder eine C1-C7-Alkylgruppe, die gegebenenfalls durch eine oder mehrere Alkoxygruppen substituiert ist,
stehen;
R₃ für eine C1-C7-Alkylgruppe steht;
R₄ für ein Wasserstoffatom oder eine C1-C4-Alkylgruppe steht;
Y für eine C1-C4-Alkoxygruppe oder eine -NRR'- oder -O(CH₂)ₙ-C(O)-NRR'- Gruppe steht, wobei R und R' die unten angegebene Bedeutung besitzen und n für eine ganze Zahl mit einem Wert von 1 oder 2 steht;
R" für eine C1-C4-Alkylgruppe steht und
R und R' unabhängig voneinander für ein Wasserstoffatom, eine -CO-C1-C4-Alkylgruppe oder eine -COOR'' -Gruppe stehen, wobei R'' die oben angegebene Bedeutung besitzt;
in Basen- oder Säureadditionssalzform sowie ein Enantiomer oder ein Diastereoisomer einschließlich eines Gemischs davon.

2. Verbindungen nach Anspruch 1, für die Y für eine C1-C4-Alkoxygruppe steht,
in Basen- oder Säureadditionssalzform sowie ein Enantiomer oder ein Diastereoisomer einschließlich eines Gemischs davon.

3. Verbindungen nach Anspruch 1, für die Y für eine -NRR'-Gruppe steht, wobei R und R' die in Anspruch 1 angegebene Bedeutung besitzen,
in Basen- oder Säureadditionssalzform sowie ein Enantiomer oder ein Diastereoisomer einschließlich eines Gemischs davon.

4. Verbindungen nach Anspruch 1, für die Y für eine -O(CH₂)n-C(O)-NRR'-Gruppe steht, wobei R und R' die in Anspruch 1 angegebene Bedeutung besitzen und n für eine ganze Zahl mit einem Wert von 1 oder 2 steht,
in Basen- oder Säureadditionssalzform sowie ein Enantiomer oder ein Diastereoisomer einschließlich eines Gemischs davon.

5. Verbindungen nach Anspruch 1, **dadurch gekennzeichnet, daß** sie unter
■ {3-[7-Amino-8-ethyl-6-(methylcarbamoyl)-5-oxo-5,8-dihydro-1,8-naphthyridin-2-yl]prop-2-in-1-yl}-carbamidsäuremethylester
■ 2-Amino-7-(3-amino-3-methylbut-1-in-1-yl)-1-ethyl-N-methyl-4-oxo-1,4-dihydro-1,8-naphthyridin-3-carboxamid-hydrochlorid
■ (±)-2-Amino-7-(3,4-dimethoxy-3-methylbut-1-in-1-yl)-1-ethyl-N-methyl-4-oxo-1,4-dihydro-1,8-naphthyridin-3-carboxamid
■ 2-Amino-7-[3-(2-amino-2-oxoethoxy)-3-methylbut-1-in-1-yl]-1-ethyl-N-methyl-4-oxo-1,4-dihydro-1,8-naphthyridin-3-carboxamid
■ 2-Amino-1-ethyl-7-(3-methoxyprop-1-in-1-yl)-N-methyl-4-oxo-1,4-dihydro-1,8-naphthyridin-3-carboxamid
ausgewählt sind.

6. Verfahren zur Herstellung einer Verbindung der Formel (I) nach einem der Ansprüche 1 bis 5, **dadurch gekennzeichnet, daß** man eine Verbindung der Formel (VII) 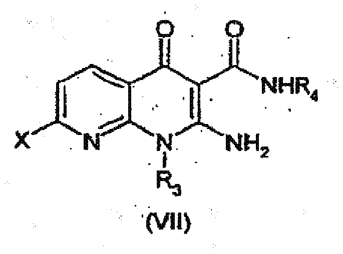 worin X für ein Halogenatom steht und R₃ und R₄ die in einem der vorhergehenden Ansprüche angegebene Bedeutung besitzen,
mit einer Verbindung der Formel (VIII) 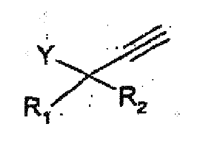 worin Y, R₁ und R₂ die in einem der vorhergehenden Ansprüche angegebene Bedeutung besitzen,
umsetzt.

7. Medikament, **dadurch gekennzeichnet, daß** es eine Verbindung der Formel (I) nach einem der Ansprüche 1 bis 5 oder ein Additionssalz dieser Verbindung mit einer pharmazeutisch unbedenklichen Säure oder auch ein Enantiomer oder ein Diastereoisomer der Verbindung der Formel (I) oder ein Gemisch davon umfaßt.

8. Pharmazeutische Zusammensetzung, **dadurch gekennzeichnet, daß** sie eine Verbindung der Formel (I) nach einem der Ansprüche 1 bis 5 oder ein pharmazeutisch unbedenkliches Salz oder auch ein Enantiomer oder ein Diastereoisomer dieser Verbindung oder ein Gemisch davon sowie mindestens einen pharmazeutisch unbedenklichen Hilfsstoff umfaßt.

9. Kombination von mindestens einer Verbindung der Formel (I) nach einem der Ansprüche 1 bis 5 mit mindestens einem Chemotherapiemittel, das unter:
● Alkylierungsmitteln,
● Interkalationsmitteln,
● Antimikrotubulusmitteln,
● Antimitotika,
● Antimetaboliten,
● Antiproliferationsmitteln,
● Antibiotika,
● Immunmodulatoren,
● Antiphlogistika,
● Kinaseinhibitoren,
● antiangiogonen Mitteln,
● antivaskulären Mitteln und
● östrogenen und androgenen Hormonen
ausgewählt ist.

10. Verwendung einer Verbindung der Formel (I) nach einem der Ansprüche 1 bis 5 zur Herstellung eines Medikaments zur Behandlung einer Erkrankung, an der VEGFR-3 beteiligt ist.

11. Verwendung einer Verbindung der Formel (I) nach einem der Ansprüche 1 bis 5 zur Herstellung eines Medikaments zur Behandlung und/oder Prävention von Krebserkrankungen und Metastasen davon.

12. Verwendung einer Verbindung der Formel (I) nach Anspruch 11 zur Herstellung eines Medikaments zur Behandlung und/oder Prävention von Glioblastomen, multiplen Myelomen, myelodysplasischen Syndromen, Kaposi-Sarkomen, kutanen Angiosarkomen, soliden Tumoren, Lymphomen, Melanomen, Brustkrebserkrankungen, Kolorektalkrebserkrankungen, Lungenkrebserkrankungen einschließlich nichtkleinzelligen Krebserkrankungen, Bauchspeicheldrüsenkrebserkrankungen, Prostatakrebserkrankungen, Nierenkrebserkrankungen, Kopf- und Halskrebserkrankungen, Leberkrebserkrankungen, Eierstockkrebserkrankungen, Krebserkrankungen der Atemwege und des Thorax und anderen Tumoren, die VEGFR-3 exprimieren oder an denen ein Angiogenese- oder Lymphangiogeneseprozeß beteiligt ist.

13. Verwendung einer Verbindung der Formel (I) nach einem der Ansprüche 1 bis 5 zur Herstellung eines Medikaments zur Behandlung und/oder Prävention von nichtonkologischen proliferativen Erkrankungen und pathologischen Angiogenesen, die mit VEGFR-3 in Verbindung stehen.

14. Verwendung einer Verbindung der Formel (I) nach Anspruch 13 zur Herstellung eines Medikaments zur Behandlung und/oder Prävention von Erkrankungen wie Arthrosen, Restenosen, Psoriasis, Hämangiomen, Lymphangiomen, Glaukomen, Glomerulonephritiden, diabetischen Nephropathien, Nephrosklerosen, thrombotischen mikroangiopathischen Syndromen, Leberzirrhosen, Atherosklerosen, Organtransplantatabstoßung und Augenerkrankungen, an denen ein Angiogenese- oder Lymphangiogeneseprozeß beteiligt ist.

15. Verwendung einer Verbindung der Formel (I) nach einem der Ansprüche 1 bis 5 zur Herstellung eines Medikaments zur Behandlung und/oder Prävention von chronischer oder nichtchronischer Entzündung, Infektion mit Mikroorganismen und Autoimmunerkrankungen, wie rheumatoider Arthritis.

16. Verwendung einer Verbindung der Formel (I) nach einem der Ansprüche 1 bis 5 zur Herstellung eines Medikaments zur Behandlung und/oder Prävention von seltenen Erkrankungen wie Lymphangioleiomyomatose.
